# EUROPEAN PATENT SPECIFICATION

(11) **EP 2 553 444 B1**
(45) Date of publication and mention of the grant of the patent: **26.09.2018**
(21) Application number: 11713951.9
(22) Date of filing: 31.03.2011
(51) Int. Cl.: G01N 33/00, A61K 8/37, A61K 8/368, A61K 8/35, A61K 8/33, A61K 8/34, A61K 8/41, A61K 8/49, A61Q 11/00

(54) **WHOLE MOUTH MALODOR CONTROL BY A COMBINATION OF ANTIBACTERIAL AND DEODORIZING AGENTS**
BEKÄMPFUNG VON MUNDGERUCH MIT EINER KOMBINATION AUS ANTIBAKTERIELLEN UND DESODORIERENDEN MITTELN
CONTRÔLE TOTAL DE LA MAUVAISE HALEINE PAR UNE COMBINAISON D'AGENTS ANTIBACTÉRIENS ET DÉSODORISANTS

(30) Priority: 31.01.2011 US 437815 P; 01.04.2010 US 319897 P
(43) Date of publication of application: 06.02.2013
(73) Proprietor: The Procter & Gamble Company, Cincinnati, OH 45202 (US)
(72) Inventor: RAMJI, Niranjan, Mason, Ohio 45040 (US); SNIDER, Ann, Gilligan, Loveland, Ohio 45140 (US); WITTE, Lina, Aurora, Monroe, Ohio 45050 (US); BECKER, Beverly, D., Milford, Ohio 45150 (US); STATT, Beth, Hansell, Lebanon, Ohio 45036 (US); NOLAND, Andrea, L., West Chester, Ohio 45069 (US); MCKINNEY, Kristi, Liberty Township, Ohio 45044 (US)
(74) Representative: Joos, Uta Susanne
(86) International application number: PCT/US2011/030665
(87) International publication number: WO 2011/123601

(56) References cited:
- WO-A1-2004/073671
- WO-A1-2006/032666
- WO-A1-2007/025401
- WO-A2-2008/005548
- US-A1- 2003 165 439
- Laverne D. Small ET AL: "The determination of carvone in oil of spearmint", Journal of the American Pharmaceutical Association, vol. 41, no. 5, 23 May 1952 (1952-05-23), pages 280-282, XP55249089, ISSN: 0095-9553, DOI: 10.1002/jps.3030410519

## Description

### CROSS REFERENCE TO RELATED APPLICATIONS

This application claims the benefit of U.S. Provisional Application No. 61/319,897, filed April 1, 2010 and U.S. Provisional Application No. 61/437,815, filed January 31, 2011.

### TECHNICAL FIELD

The present invention relates to oral care compositions containing a metal ion source antibacterial agent and anisaldehyse and β-ionone as deodorizing or odor neutralizing agents to provide whole mouth malodor control.

### BACKGROUND OF THE INVENTION

Oral malodor, also called halitosis or bad breath, is a universally experienced condition that has a variety of etiologic factors. The condition may have many causes including prolonged fasting, oral pathology such as gingivitis or periodontitis, and otolaryngologic sources such as sinusitis. However, even individuals without oral or nonoral pathology experience periodic breath malodor, which may persist for hours (or even days) after ingesting foods containing certain odoriferous items, such as garlic and onion. Although numerous non-oral sites and many different causes have been correlated to bad breath, it has been estimated that 80 percent to 90 percent of all bad breath odors originate from the mouth, and bacteria are directly responsible for most of the offensive gases. Putrefaction by oral bacteria of sulfur-containing proteins and amino acids to sulfur gases is postulated to be the source of breath malodor in most instances where it has been studied [J. Periodontol. (1977), 48:13-20]. Odor-producing sulfur compounds detected in individuals with halitosis include hydrogen sulfide, methyl mercaptan, dimethyl sulfide, dimethyl disulfide and ethanethiol *[*Clin. Chim. Acta (1978), 85:279-284]. Oral environments that promote generation of these compounds include those low in carbohydrates, with a neutral or alkaline pH, and which are anaerobic (J. Periodontol. (1992), 63:768-775].

The oral cavity has numerous micro environments to harbor bacteria and contribute to oral malodor. Intraoral conditions such as poor oral hygiene, a tongue coating, extraction sites, necrotic tissues, carious lesions, open contacts allowing food impaction, prosthetic appliances, ill-fitting restorations, and inflammatory lesions of gingivitis and periodontitis contribute to oral malodors. There is evidence to suggest that oral malodor is caused by the microorganisms that cause gingivitis and periodontitis (i.e., periodontal pathogens). In the presence of adequate substrate with appropriate conditions, a sequence of events leads to the release into the oral cavity of odoriferous gases that pollute exhaled air and are perceived as bad breath. Research has identified several microorganisms responsible for producing these offensive odors and the conditions necessary for their production. In addition to the presence of certain types of bacteria, the type and amount of substrate as well as oxygen, saliva and pH levels have been found to influence the occurrence and severity of oral malodor.

Certain chemical end products of bacterial putrefaction referred to as volatile sulfur compounds (VSC's) are foul-smelling and determined to be responsible for the offensive odor recognized as bad breath. Nonsulfur-containing compounds such as cadaverine, putrescine, indole and skatole have also been implicated in the foul smell of oral malodor, but their contribution is thought to be limited. VSC's such as hydrogen sulfide, methyl mercaptan, dimethyl sulfide and dimethyl disulfide make up more than 90 percent of the foul odors from the mouth, with hydrogen sulfide and methyl mercaptan accounting for approximately 90 percent of the total VSC's. Specific groups of bacteria responsible for the production of oral malodor have been identified in reported studies [T.F. McNamara, et al., Oral Surg Oral Med. Oral Pathol. (1972), 34(1):41-8; J. Tonzetich, J. Periodontol. (1977), 48(1):13-20]. It was demonstrated that the formation of foul odors from incubated saliva correlated with a shift in the microbial flora from a predominately gram-positive to a predominately gram-negative anaerobic flora. Concomitant with this shift in flora, a depletion of carbohydrates and a rise in the pH level of the stagnant saliva were observed. As the carbohydrates are depleted, the gram-positive acidogenic flora are suppressed and the gram-negative microorganisms, capable of protein metabolism, become progressively more dominant. Evaluated individually, none of nine gram-positive microorganisms produced an unpleasant odor whereas all four of gram-negative microorganisms (*Fusobacterium polymorphum, Veillonella alcalescens, Bacteroides fundiliformis, Klebsiella pneumoniae*) produced a foul odor. A similar study found that gram-negative microorganisms (*Veillonella alcalescens, Fusobacterium nucleatum, Bacteroides melanogenicus* and *Klebsiella pneumoniae*) produced VSC's [M.C. Solis-Gaffar, et al., J Soc. Cosmet. Chem. (1979), 30:241-7]. Production of VSC's was accompanied by an unpleasant odor and a rise in the pH level of the saliva mixture.

Anaerobic conditions, necessary for the production of VSC's, are created in microenvironments. The accumulation of plaque and debris and the stagnation of saliva occur most commonly in areas where tooth and tissue crevices lend themselves to stagnant microenvironments. Most common sites for stagnation, plaque accumulation and production of VSC's include the posterior dorsum tongue, interdental spaces and subgingival areas. Dental plaque progresses from an aerobic, gram-positive colonization to one that is anaerobic, favoring gram-negative growth. Anaerobic bacteria produce foul-smelling VSC's from sulfur-containing precursor substrates, such as sulfur-containing peptides and amino acids found in saliva and crevicular fluid. Proteinaceous substrates such as exfoliated epithelium, leukocytes, food debris and dead bacteria, are degraded by proteolysis into peptides and amino acids. The sulfur-containing amino acids, specifically cysteine and methionine, are further degraded into VSC's. Gas chromatographic studies have identified hydrogen sulfide and methyl mercaptan as the putrefactive end products of cysteine and methionine, respectively. The VSC level and severity of oral malodor are influenced by bacterial plaque levels, substrate availability, time of day and oral dryness. Oral malodor is worst in the morning, following an extended period of oral dryness and undisturbed bacterial putrefaction, thus the common term "morning breath" which is synonymous with bad breath or halitosis.

Oral malodor after consuming foods such as garlic and onion are also attributed to VSC's consisting of a mixture of thiols, sulfides and disulfides. Odoriferous compounds in garlic include hydrogen sulfide, methyl mercaptan, allyl disulfide, allyl methyl sulfide, allyl methyl disulfide and allyl mercaptan [F. Suarez, et al., Am. J. Physiol. Gastrointest. Liver Physiol. (1999), 276: G425-G430]. Of these, allyl mercaptan is the most stinky or foul-smelling, having the lowest odor threshold. The Suarez study reported one gas in particular, allyl methyl sulfide, was exhaled for prolonged periods after garlic ingestion as a result of gastrointestinal absorption, systemic circulation, and ultimate excretion from the lungs. Data from this study explain the tenacious nature of garlic breath; even aggressive oral hygiene did not prevent the expulsion of allyl methyl sulfide in the breath of subjects who consumed garlic. U.S. Patent Applications 2003/165439A1 discloses that the combination of cetylpyridium chloride, zinc acetate and alpha-ionone provides a synergistic antibacterial efficacy against representative oral bacteria which are implicated in the production of mouth malodor. WO2004/073671A1 discloses a flavoured product comprising four or more flavor materials having antimicrobial properties comprising inter alial anisaldehyde and cationic salts. WO2006/032666A1 discloses to use eugenol acetate for inhibiting and/or preventing the growth of and/or for killing microorganisms which cause bad breath. Eugenol acetate shall be combined with eugenol and an aroma composition comprising menthol, an aniseed note, eucalyptol or herbal components.

Since oral malodor is a multifactorial problem, successful treatment or control needs to address all contributing factors. The present invention thus provides oral care products comprising a combination of active agents to control odors produced by anaerobic bacteria (such as morning breath) as well as non-bacterial odors such as derived from consumption of certain foods, beverages and medicines. In one aspect, the oral care products comprise an antibacterial agent effective at eliminating or reducing the causative microorganisms and their capability to produce VSC's and a neutralizing or deodorizing agent which functions to bind VSC's rendering them nonvolatile and non-odoriferous.

### SUMMARY OF THE INVENTION

The present invention is directed to oral care compositions comprising in a pharmaceutically acceptable carrier, a combination of a metal ion source antibacterial agent and 4-methoxybenzaldehyde (anisaldehyde) and beta-ionone as deodorizing or odor-neutralizing agent comprising a compound having the structure 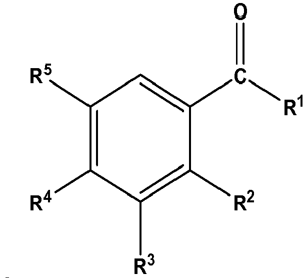 wherein R¹, R², R³, and R⁵ may be identical or different, each representing H, linear or branched C1-C6 alkyl or alkenyl, phenyl, -OH or -OR^{a}; R⁴ is -OH, -OR^{a}, phenyl, or linear or branched C1-C6 alkyl or alkenyl; and R^{a} is phenyl or linear or branched C1-C6 alkyl or alkenyl. The antibacterial agent comprise a metal ion source to supply metal ions such as stannous, zinc or copper. The present compositions provide effective control of mouth and breath malodor.

In a further aspect the present invention provides for a method and kit for demonstrating and comparing the effectiveness of oral care compositions such as mouthrinses and dentifrices for controlling mouth malodor such as associated with consumption of garlic and onion.

These and other features, aspects, and advantages of the present invention will become evident to those skilled in the art from the detailed description which follows.

### DETAILED DESCRIPTION OF THE INVENTION

While the specification concludes with claims particularly pointing out and distinctly claiming the invention, it is believed that the present invention will be better understood from the following description.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated. All measurements referred to herein are made at 25°C unless otherwise specified.

Herein, "comprising" means that other steps and other components which do not affect the end result can be added. This term encompasses the terms "consisting of" and "consisting essentially of."

As used herein, the word "include," and its variants, are intended to be non-limiting, such that recitation of items in a list is not to the exclusion of other like items that may also be useful in the materials, compositions, devices, and methods of this invention.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

By "oral care composition" is meant a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouthrinse, mousse, foam, denture product, mouthspray, lozenge, chewable tablet or chewing gum. The oral care composition may also be incorporated onto strips or films for direct application or attachment to oral surfaces.

The term "dentifrice", as used herein, means paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having the gel surrounding the paste, or any combination thereof. Each dentifrice composition in a dentifrice comprising two or more separate dentifrice compositions may be contained in a physically separated compartment of a dispenser and dispensed side-by-side.

The term "dispenser", as used herein, means any pump, tube, or container suitable for dispensing compositions such as dentifrices.

The term "teeth" refers to natural teeth as well as artificial teeth or dental prosthesis.

The terms "pharmaceutically acceptable carrier", "orally acceptable carrier" or "excipients" include safe and effective materials and conventional additives such as those used in oral care compositions including but not limited to fluoride ion sources, antibacterial agents, anticalculus or anti-tartar agents, peroxide sources, abrasives such as silica, buffering agents, alkali metal bicarbonate salts, thickening materials, humectants, water, surfactants, titanium dioxide, flavor systems, sweetening agents, xylitol, and coloring agents.

The term "essential oils" as used herein refers to volatile oils distilled or expressed from plants and constituents of these volatile oils. Typical essential oils and their main constituents are those obtained for example from thyme (thymol, carvacrol), oregano (carvacrol, terpenes), lemon (limonene, terpinene, phellandrene, pinene, citral), lemongrass (citral, methylheptenone, citronellal, geraniol), orange flower (linalool, β-pinene, limonene), orange (limonene, citral), anise (anethole, safrol), clove (eugenol, eugenyl acetate, caryophyllene), rose (geraniol, citronellol), rosemary (borneol, bornyl esters, camphor), geranium (geraniol, citronellol, linalool), lavender (linalyl acetate, linalool), citronella (geraniol, citronellol, citronellal, camphene), eucalyptus (eucalyptol); peppermint (menthol, menthyl esters), spearmint (carvone, limonene, pinene); wintergreen (methyl salicylate), camphor (safrole, acetaldehyde, camphor), bay (eugenol, myrcene, chavicol), cinnamon (cinnamaldehyde, cinnamyl acetate, eugenol), tea tree (terpinen-4-ol, cineole), and cedar leaf (a-thujone, β-thujone, fenchone). Essential oils are widely used in perfumery and as flavorings, medicine and solvents [See Kirk-Othmer Encyclopedia of Chemical Technology, 4th Edition and in The Merck Index, 13th Edition].

Active and other ingredients useful herein may be categorized or described by their cosmetic and/or therapeutic benefit or their postulated mode of action or function. However, the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed.

In one embodiment of the present invention, oral care compositions for whole mouth deodorization are provided comprising an antibacterial agent comprising one or a mixture of a stannous ion source; a zinc ion source or a copper ion source in combination with a deodorizing or odor-neutralizing agent comprising a combination of 4-methoxybenzaldehyde (anisaldehyde) and β-ionone, a compound having the structure 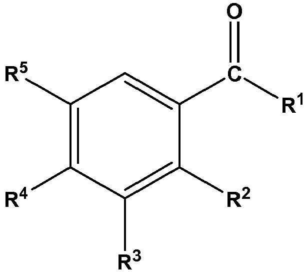 wherein R¹, R², R³, and R⁵ may be identical or different, each representing H, linear or branched C1-C6 alkyl or alkenyl, phenyl, -OH or -OR^{a}; R⁴ is -OH, -OR^{a}, phenyl or linear or branched C1-C6 alkyl or alkenyl; R^{a} is phenyl or linear or branched C1-C6 alkyl or alkenyl. Among odor-neutralizing compounds useful herein are aromatic aldehydes, ketones and carboxylic acids with a substituent (R⁴) *para* to the carbonyl group.

The odor-neutralizing or deodorization activity of target actives was evaluated against a recreated garlic odor using an *in-vitro* headspace gas chromatography-mass spectrometry (GC-MS) method described below. A mixture of five sulfur compounds (ethanethiol, 1-propanethiol, dimethyl disulfide, methyl allyl disulfide and dimethyl disulfide) was used to simulate a garlic odor. The mixture includes sulfur compounds that have been reported among malodorous components having the characteristic odor of garlic and present in human breath after consumption of garlic. [See e.g., J. Taucher, et al. J. Agric. Food Chem. (1996), 44 (12), 3778-3782; F. Suarez, et al. Am. J. Physiol. Gastrointest. Liver Physiol. (1999), 276: G425-G430; I. Laakso, et al. Planta Med. (1989), 55, 257-261; X. J. Cai,et al. J. Agric. Food Chem. (1995), 43, 1751-1753; T. Minami, et al. J. Food Sci. (1989), 54, 763-765.]

The following procedure and instrumentation were used to evaluate the relative odor-neutralizing activity of various compounds.
1) Purge product base (e.g., mouthrinse or dental solution) with nitrogen for 10 minutes.
2) Purge all sample preparation vials with stir bars inside for 1 minute.
3) Add 3960 µl product base to control vials (4 ml capacity) and 3560 µl product base to test sample vials (4 ml capacity).
4) Make garlic stock by mixing the following components in order: 600 µl ethanol; 500 µl ethanethiol; 80 µl 1-propanethiol; 30 µl dimethyl disulfide; 20 µl methyl allyl disulfide;70 µl dimethyl disulfide.
5) Add 40ul of garlic stock to each control and test sample vial.
6) Stir all samples to mix product base and garlic stock for a total time of 20 min. To each test sample vial, add 400 µl of test active after 15 min. of stirring and allow test active mix in and react for 5 min.
7) After mixing, transfer 50 µl each of control sample and test sample to nitrogen purged GC headspace vials.
8) Place control and test sample vials on headspace autosampler for GC analysis. Each sample is equilibrated at 32 °C for 3 min. before injection into the GC instrument.

**GC Parameters**

| | |
|---|---|
| Column | HP-1MS Agilent 19091s-733 Length: 30m ID: 0.25mm Thickness: 1um |
| Temperature | 35°C Hold 4 min ; 90°C / min to 250°C Hold 2 min ; |
| Run Time | 8.39 min |
| Flow | 1.6 ml / min |
| Split Ratio | 5.0 : 1 |
| Autos ampler | Agilent HP7694 |

**Autosampler Parameters**

| Temperature Setpoints | |
|---|---|
| Oven | 32°C |
| Sample Valve | 115°C |
| Transfer Line | 120°C |

| Timing Parameters | |
|---|---|
| GC Cycle | 16.5 min |
| Vial Equilibration | 3.0 min |
| Vial Pressurization | 0.5 min |
| Sample Loop Fill | 0.5 min |
| Loop Equilibration | 0.10 min |
| Sample Injection | 1.00 min |
| Shaking | Low |

Corresponding peak areas for each of the five component compounds making up the garlic odor stock were obtained for the control and test active samples and used along with odor threshold values of the compounds to calculate a Relative Odor Activity Value (ROAV). A Relative OAV is calculated for each component by dividing its peak area by its odor threshold value. Odor threshold values for these compounds are reported in the literature. (See e.g., Leffingwell & Associates, "Odor & Flavor Detection Thresholds in Water"; http://www.lenntech.com/table.htm) An average ROAV for a control or test active sample is calculated from the ROAV's of the five components. The difference between the average ROAV of the control and the average ROAV of the test active sample measures the reduction of garlic odor achieved by the test active. Test actives that neutralize garlic odor would have a lower ROAV than control. The larger the difference, the more potent the odor neutralizing activity of the test active. Results of testing various compounds are shown below as % reduction of ROAV. A negative value indicates no reduction in ROAV.

| Test Active | % Reduction of ROAV |
|---|---|
| Anisaldehyde | 20.76 |
| Cinnamaldehyde | 10.73 |
| β-Ionone | 17.56 |
| Citral | -19.54 |
| *para*-Methoxycinnamaldehyde | -0.49 |
| Octanal | -28.76 |
| Salicylaldehyde | -5.87 |
| Benzaldehyde | -0.75 |
| Heliotropin | -2.92 |
| 4-methylbenzaldehyde | 3.67 |
| Ethyl vanillin | 4.02 |
| Methyl vanillin (Veratraldehyde) | 4.45 |
| Hexyl cinnamaldehyde | -1.19 |
| Ethyl maltol | -3.46 |
| L(-) Carvone | 3.27 |
| (-) Piperitone | 10.19 |

This data set was used to develop a structural activity relationship (SAR) model correlating the observed reduction of ROAV to specific chemical structural features of test compounds, such as quantum mechanical, physical and size-intensive properties. The best regression equation was determined and further used to predict % reduction of ROAV for other chemical ingredients. In order to develop the QSAR (quantitative structural activity relationship), the % reduction of OAV as determined by the above experiment at 0.05% active concentration was used in the CaChe 7.1 molecular modeling programme from Fujitsu Limited. The % Reduction of OAV from a training set of 16 chemical samples preconditioned with PM5 was used to calculate the Complete Quantum QSAR. The CAChe MOPAC (Molecular Orbital Package) application determines both an optimum geometry and the electronic properties of molecules by solving the Schrödinger equation using the semi-empirical Hamiltonians AM1, PM3 and PM5, developed by M. J. S. Dewar and J. J. P. Stewart. [See J. Am. Chem. Soc. (1985),107, 3902; J. Comput. Chem. (1989),10, 209; MOPAC 2002, (1999),Fujitsu Limited]

Based on this model, compounds that would provide desired odor neutralizing activity, i.e., a % reduction of ROAV of at least about 1, include those with the chemical structure above, i.e., aromatic carbonyl compounds such as aldehydes, ketones, acids and esters with the carbonyl group directly bonded to the aromatic ring.

For example, anisaldehyde (4-methoxybenzaldehyde C₈H₈O₂) provided a 20.76% reduction of ROAV and excellent deodorizing activity, while a non-aromatic aldehyde, such as octanal (C₈H₁₆O) had no reduction of ROAV and no deodorizing activity. In addition to the aromatic carbonyl group, a structural feature found to affect activity is the substitution pattern on
the aromatic ring, i.e., the chemical nature, position and number of substituents. For example, high activity was generally found for compounds with the only ring substituent being a hydroxyl, alkoxy, alkyl or phenyl group *para* to the aromatic carbonyl. The data below demonstrate the effect of ring substituents on the measured or calculated % Reduction of ROAV.

The compounds useful as deodorizing agent are those providing at least about a 4.0 % reduction of ROAV according to this model, or those providing at least about a 6.0 % reduction. For example, anisaldehyde shown to provide better than 20% reduction of ROAV and potent deodorizing activity is useful herein. At least about a 4.0 % reduction of ROAV, include 4-isopropylbenzaldehyde (cuminaldehyde); 1-(4-methoxy-phenyl)-2-methyl-propan-1-one; 1-(4-methoxy-phenyl)-ethanone; 1-(4-methoxy-phenyl)-propan-1-one; 1-(4-Methoxy-phenyl)-butan-1-one; 4-isobutylbenzaldehyde; 4-propylbenzaldehyde; 4-butylbenzaldehyde; 2,4-dimethyl benzaldehyde; 2,4,5 trimethyl benzaldehyde; 4-phenylbenzaldehyde; 4-methoxybenzoic acid and 4-methoxybenzoic acid methyl ester. Because of the potent deodorizing activity, the present deodorizing agents may be used in the present compositions at low levels of from about 0.01% to about 0.2% by weight of the composition, from about 0.01% to about 0.1% in some embodiments, such as about 0.03%, about 0.05% or about 0.075%.

| Compound | % Reduction of ROAV |
|---|---|
| 4-Methoxybenzaldehyde (Anisaldehyde) | 20.76 |
| 4-Isopropoxybenzaldehyde | 10.49 |
| 4-Isobutoxybenzaldehyde | 9.43 |
| 4-Ethoxybenzaldehyde | 9.08 |
| 4-Propoxybenzaldehyde | 8.66 |
| 4-Butoxybenzaldehyde | 7.82 |
| 4-Phenoxybenzaldehyde | 3.25 |
| 3,4-Dimethoxybenzaldehyde (Methyl vanillin) | 4.45 |
| 3-Methoxybenzaldehyde | 4.27 |
| 2-Ethoxy-4-hydroxybenzaldehyde (Ethyl vanillin) | 4.02 |
| 4-Hydroxybenzaldehyde | 5.67 |
| 4-Hydroxy-3-methoxybenzaldehyde (Vanillin) | 1.96 |
| 2,3-Dimethoxybenzaldehyde | 1.20 |
| 2-Methoxybenzaldehyde | 0 |
| 2-Hydroxybenzaldehyde | 0 |
| 4-Isopropylbenzaldehyde (Cuminaldehvde) | 11.45 |
| 4-Isobutylbenzaldehyde | 10.89 |
| 4-Propylbenzaldehyde | 10.03 |
| 4-Butylbenzaldehyde | 9.35 |
| 4-Ethylbenzaldehyde | 8.90 |
| 4-methyl benzaldehyde | 3.67 |
| 2,4-dimethyl benzaldehyde | 6.28 |
| 2,4,5 trimethyl benzaldehyde | 9.87 |
| 4-Phenylbenzaldehyde | 4.46 |
| 4-Methylbenzaldehyde | 3.67 |
| 1-(4-Methoxy-phenyl)-2-methyl-propan-1-one | 14.06 |
| 1-(4-Methoxy-phenyl)-ethanone | 10.33 |
| 1-(4-Methoxy-phenyl)-propan-1-one | 10.29 |
| 1-(4-Methoxy-phenyl)-butan-1-one | 9.66 |
| Bis-(4-methoxy-phenyl)-methanone | 4.24 |
| 1-(4-Methoxy-phenyl)-phenyl-methanone | 2.62 |
| 4-Methoxybenzoic acid methyl ester | 9.65 |
| 4-Methoxybenzoic acid | 6.58 |

The carbonyl compounds that have potent deodorization activity are believed to react with the malodorous volatile sulfur compounds (VSC's) forming species that are less volatile and thus, less odoriferous. As shown in the following reaction scheme, thiol compounds and to a smaller extent sulfide compounds can react with the carbonyl compound, such as an aldehyde or ketone to form thioacetals or thioketals, which are not very volatile thereby resulting in a reduction in the perceived odor. The stronger the Lewis acid (R") in this reaction scheme, the more susceptible the carbonyl carbon becomes to nucleophilic attack by the thiol (RSH) and the greater is the conversion of the aldehyde/ketone to the corresponding thioacetal/thioketal. 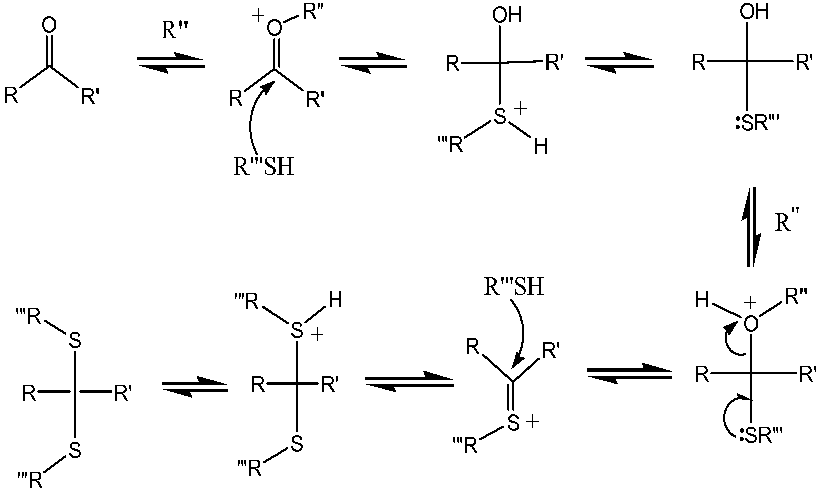 R" = H⁺, Lewis acid

The odor-neutralizing agent may further comprise an additional odor-neutralizing compound including β-ionone, . These additional odor-neutralizing compounds may be used at a level of from about 0.01% to about 0.1% by weight of the composition. A number of these odor-neutralizing compounds are flavor chemicals and may be added in the composition as part of the flavor system.

A second component of the present compositions for effective whole mouth deodorization is an antimicrobial agent, in particular an antibacterial agent to provide effectiveness in killing, and/or altering metabolism, and/or suppressing the growth of, microorganisms which cause topically-treatable infections and diseases of the oral cavity, such as plaque, caries, gingivitis, and periodontal disease. The orally-effective antibacterial agents are metal ions such as stannous, zinc and copper; and mixtures thereof. The level of antibacterial agent is dependent on the type of antibacterial agent and other factors and is from about 0.01% to about 5.0%, by weight of the composition.

The quaternary ammonium compounds include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethoylstearyl ammonium chloride, cetylpyridinium chloride, quaternized 5-amino-1,3-bis(2-ethyl-hexyl)-5-methyl hexa hydropyrimidine, benzalkonium chloride, benzethonium chloride and methyl benzethonium chloride are exemplary of typical quaternary ammonium antibacterial agents. Other compounds are bis[4-(R-amino)-1-pyridinium] alkanes as disclosed in U.S. No. 4,206,215, Jun. 3, 1980 to Bailey. The pyridinium compounds are the preferred quaternary ammonium compounds, particularly preferred being cetylpyridinium, or tetradecylpyridinium halide salts (i.e., chloride, bromide, fluoride and
iodide). Most preferred is cetylpyridinium chloride. The quaternary ammonium antimicrobial agents may be included in the present invention at levels of at least about 0.035%, from about 0.045% to about 1.0%, or from about 0.05% to about 0.10% by weight of the composition.

The present compositions comprise a metal ion source that provides stannous ions, zinc ions, copper ions, or mixtures thereof as antibacterial agent. The metal ion source can be a soluble or a sparingly soluble compound of stannous, zinc, or copper with inorganic or organic counter ions. Examples include the fluoride, chloride, chlorofluoride, acetate, hexafluorozirconate, sulfate, tartrate, gluconate, citrate, malate, glycinate, pyrophosphate, metaphosphate, oxalate, phosphate, carbonate salts and oxides of stannous, zinc, and copper.

Stannous, zinc and copper ions have been found to help in the reduction of gingivitis, plaque, sensitivity, and improved breath benefits. The composition may comprise from about 50 ppm to about 20,000 ppm metal ion of the total composition, from about 500 ppm to about 15,000 ppm or from about 3,000 ppm to about 10,000 ppm. This is the total amount of metal ions (stannous, zinc, copper and mixtures thereof) for delivery to the tooth surface.

Dentifrices containing stannous salts, such as stannous fluoride and stannous chloride, are described in U.S. Patent 5,004,597 to Majeti et al. Other descriptions of stannous salts are found in U.S. Patent 5,578,293 issued to Prencipe et al. and in U.S. Patent 5,281,410 issued to Lukacovic et al. In addition to the stannous ion source, ingredients needed to stabilize the stannous may be included, such as those described in Majeti et al. and Prencipe et al.

Stannous salts useful herein include stannous fluoride and stannous chloride dihydrate, stannous acetate, stannous tartrate and sodium stannous citrate. Examples of suitable zinc ion sources are zinc oxide, zinc sulfate, zinc chloride, zinc citrate, zinc lactate, zinc gluconate, zinc malate, zinc tartrate, zinc carbonate, zinc phosphate, and other salts listed in U.S. Pat. No 4,022,880. Examples of suitable copper ion sources are listed in U.S. Pat. No. 5,534,243 and include the chloride, sulfate and glycinate salts. The combined metal ion source(s) may be be present in an amount of from about 1% to about 5 % by weight of the final composition. The stannous salts will typically be present in an amount of from about 1% to about 5%, or from about 1.5% to about 3% by weight of the total composition. The amount of zinc or copper salts used in the present invention typically ranges from about 0.01 to about 5%, from about 0.05 to about 4%, or from about 0.1 to about 3.0%.

Antibacterially-effective essential oils include one or more of flavor/fragrance chemicals such as citral, neral, geranial, geraniol, nerol, eucalyptol, eugenol, carvacrol, thymol, o-cymen-5-ol (isopropylmethylphenol, IPMP), farnesol, benzyl alcohol, benzaldehyde, hinokitiol (isopropyltropolone), terpinene-4-ol, zingerone, allyl isothiocyanate, dipentene, α-pinene, β-pinene, menthol, methyl salicylate, anethole, carvone, limonene, ocimene, n-decyl alcohol, citronellal, citronellol, methyl acetate, citronellyl acetate, methyl eugenol, linalool, ethyl linalool, camphor, safrole, chlorothymol, guaiacol, phenol, phenyl salicylate , cinnamic acid, guaiacol, isoeugenol, dihydroeugenol, vanillyl butyl ether, 5-propenylguaethol, 4-ethyl-2-methoxyphenol, 4-allyl-2-methoxyphenol acetate, and 4-methyl guaiacol. Natural sources of these chemicals may be used. The selection of the essential oils to is based on demonstration of their activity against microorganisms known to be involved in undesirable oral cavity conditions such as gingivitis, periodontal disease and oral malodor. For example, useful herein is a blend of essential oils comprising at least two components, a first component selected from acyclic or non-ring structures including citral, neral, geranial, geraniol, nerol or derivatives thereof and a second component selected from ring-containing structures including eucalyptol, eugenol, carvacrol or derivatives thereof, such as described in commonly-assigned patent application published as US20080253976A1. The essential oil blend is used at a level of at least about 0.02% by weight of the composition to provide effective antibacterial activity.

Antibacterial agents for use herein may comprise one or a mixture of metal ions such as stannous, zinc and copperand selected essential oil blends. Triclosan and other agents of this type are disclosed in Parran, Jr. et al., U.S. Patent 5,015,466, issued May 14, 1991, and U.S. Patent 4,894,220, Jan. 16, 1990 to Nabi et al.

In addition to the *in vitro* tests described above, studies were conducted to assess *in vivo* deodorization activity of compositions comprising an antibacterial agent, an odor-neutralizing or deodorizing agent and in combination as described below.

### A. Assessment of Deodorization of Garlic Odor in-vivo

The study design was a 3-period, 3-treatment cross over study with 5 subjects with a 1-day wash out between each period, except for a 2-day washout on the weekend.

Subjects rinsed with 20 ml of water for 30 seconds to hydrate the mouth. Subjects then chewed 500 mg of garlic on a chip for 1 minute and expectorated the residue. Baseline breath was measured by sampling 2.5 ml of mouth air with a syringe. The subjects then rinsed their mouth with 20 ml of test rinse for 30 seconds. Test rinse products were made by formulating active(s) in a 10% glycerine aqueous solution. After 25 minutes post use of test rinse the head space of the mouth was again sampled by drawing 2.5 ml of air from head space of the mouth. Both the baseline sample and the 25 minute breath samples were run on a GC with sulfur chemiluminescence detection and the peak areas of interest namely those of allylmercaptan, allylmethylsulfide, allylsulfide and allyldisulfide were evaluated. The % reduction of the peaks 20 min post use of test product over initial garlic challenge was evaluated for the four peaks. Results are shown below as geometric means of the % reduction of the 4 peaks for each of the treatments. These results demonstrate that most effective deodorization is provided by the combination of an antibacterial (Zn⁺² ions from zinc lactate) and one or more deodorizers (anisaldehyde and ionone). Zn⁺² ions also provide some ability to neutralize odors due to sulfur volatiles and are thus useful as active in the present compositions.

| Treatment | % Reduction VSC Peaks |
|---|---|
| 0.03% Anisaldehyde | 62.7% |
| 0.1% Zinc lactate | 57.01% |
| 0.1% Zinc lactate+ 0.03% anisaldehyde | 67.6% |
| 0.1% Zinc lactate +0.03% anisaldehyde +0.02% β-ionone | 76.01% |

### B. Assesment of Deodorization of Morning Mouth Breath in vivo

Volatile sulfur compounds (VSC's) in morning breath were evaluated by a Halimeter after the use of test rinse products. Germ kill was also evaluated by standard microbiological assays. Test rinse products were made by formulating active(s) in a mouthrinse base containing 0.05% cetyl pyridinium chloride (CPC).

The study was a randomized, single blind crossover study with a total of 4 periods and a 2 day washout between periods. Subjects were randomized across treatments for each period. Each period was 24 hrs from baseline (morning Day 0) to next day (morning Day 1) during which subjects treated themselves 3 times on Day 0. The study had 12 subjects. Subjects treated themselves with test rinse unsupervised three times on Day 0 (after baseline measurements, late afternoon and at bedtime) of each period. All subjects rinsed with their assigned treatments for 30 seconds and then expectorated. They were asked to refrain from eating or drinking for 30 minutes following treatment. Subjects were not allowed to brush with another toothbrush or paste, use mouthwash, floss teeth or chew gum during treatment phase of study.

Breath odor (Halimeter) and germ kill (tongue) assessments were made at baseline (morning Day 0) and final at 24 hrs (morning of Day 1). All measurements were taken in the morning. Subjects were instructed to abstain from eating, drinking, or performing any oral hygiene from bedtime the night before measurement until after breath measurements were made. Subjects were assessed for volatile sulfur compound emissions (VSC's) utilizing a commercially available portable instrument called a Halimeter (Interscan Corporation, CA). This instrument is sensitive to hydrogen sulfide and methyl mercaptan, two of the primary components of foul breath odor. Results are reported as % Reduction of these VSC's compared to water control.

For bacteria kill assessments, a microbiological tongue sample was taken from each subject by gently placing a new, clean toothbrush (Kroger child toothbrush) on the anterior third dorsal surface of the tongue, with slight agitation of the bristles on the tongue surface for 5 seconds. The brush was then transferred to a vial containing 10 ml of a microbiological transport fluid (DE broth - Dey/Engley neutralizing broth) and the brush head was clipped off into the vial. The vial was then sealed and kept cold until plated. Samples were appropriately diluted (1:10, 1:100,1:1000 and 1:10,000 final) with sterile saline and spiral plated onto TSA, ETSA-NV selective agar plates and ETSA plates to enumerate total Gram-negative anaerobes (GNA) and total facultative anaerobes (TFA) present on the tongue. Results are reported as log colony forming units (log cfu) per ml. These results showed that the combination of CPC+ Zinc + anisaldehyde + ionone provided the best deodorization benefit of the treatments. Although there was no significant difference in germ kill, i.e., reduction in anaerobic bacteria, between the CPC+Zn and CPC + Zn + anisaldehyde + ionone treatments, there was increased deodorization benefit with the latter.

| Treatment | % Reduction VSC's vs. H₂O | TFA log cfu/ml | GNA log cfu/ml |
|---|---|---|---|
| 0.05% CPC | 42.9% | 7.92 | 7.3 |
| 0.05% CPC + 0.1% Zinc lactate | 58.7% | 7.72 | 7.11 |
| 0.05% CPC + 0.1% Zinc lactate + 0.03% anisaldehyde + 0.02% β-ionone | 77.7% | 7.64 | 6.9 |

In addition to the components described above, the present compositions may comprise additional optional components collectively referred to as orally acceptable carrier materials, which are described in the following paragraphs.

### Orally Acceptable Carrier Materials

The orally acceptable carrier materials comprise one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy.

The carriers or excipients of the present invention can include the usual and conventional components of dentifrices, non-abrasive gels, subgingival gels, mouthwashes or rinses, mouth sprays, chewing gums, lozenges and breath mints as more fully described hereinafter.

The choice of a carrier to be used is basically determined by the way the composition is to be introduced into the oral cavity. Carrier materials for toothpaste, tooth gel or the like include abrasive materials, sudsing agents, binders, humectants, flavoring and sweetening agents, etc. as disclosed in e.g., U.S. Pat. No. 3,988,433 to Benedict. Carrier materials for biphasic dentifrice formulations are disclosed in U.S. Pat. Nos. 5,213,790, issued May 23, 1993, 5,145,666, issued September 8, 1992, and 5,281,410 issued January 25, 1994 all to Lukacovic et al. and in U. S. Pat. Nos. 4,849,213 and 4,528,180 to Schaeffer. Mouthwash, rinse or mouth spray carrier materials typically include water, flavoring and sweetening agents, etc., as disclosed in, e.g., U.S. Pat. No. 3,988,433 to Benedict. Lozenge carrier materials typically include a candy base; chewing gum carrier materials include a gum base, flavoring and sweetening agents, as in, e.g., U.S. Pat. No. 4,083,955 to Grabenstetter et al. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910, issued March 30, 1993 and Sept. 7, 1993, respectively both to Damani. Carriers suitable for the preparation of compositions of the present invention are well known in the art. Their selection will depend on secondary considerations like taste, cost, and shelf stability, etc.

The compositions of the present invention may also be in the form of non-abrasive gels and subgingival gels, which may be aqueous or non-aqueous. In still another aspect, the invention provides a dental implement impregnated with the present composition. The dental implement comprises an implement for contact with teeth and other tissues in the oral cavity, said implement being impregnated with the present composition. The dental implement can be impregnated fibers including dental floss or tape, chips, strips, films and polymer fibers.

In one embodiment, the compositions of the subject invention are in the form of dentifrices, such as toothpastes, tooth gels and tooth powders. Components of such toothpaste and tooth gels generally include one or more of a dental abrasive (from about 6% to about 50%), a surfactant (from about 0.5% to about 10%), a thickening agent (from about 0.1% to about 5%), a humectant (from about 10% to about 55%), a flavoring agent (from about 0.04% to about 2%), a sweetening agent (from about 0.1% to about 3%), a coloring agent (from about 0.01% to about 0.5%) and water (from about 2% to about 45%). Such toothpaste or tooth gel may also include one or more of an anticaries agent (from about 0.05% to about 0.3% as fluoride ion) and an anticalculus agent (from about 0.1% to about 13%). Tooth powders, of course, contain substantially all non-liquid components.

Other embodiments of the subject invention are liquid products, including mouthwashes or rinses, mouth sprays, dental solutions and irrigation fluids. Components of such mouthwashes and mouth sprays typically include one or more of water (from about 45% to about 95%), ethanol (from about 0% to about 25%), a humectant (from about 0% to about 50%), a surfactant (from about 0.01% to about 7%), a flavoring agent (from about 0.04% to about 2%), a sweetening agent (from about 0.1% to about 3%), and a coloring agent (from about 0.001% to about 0.5%). Such mouthwashes and mouth sprays may also include one or more of an anticaries agent (from about 0.05% to about 0.3% as fluoride ion) and an anticalculus agent (from about 0.1% to about 3%). Components of dental solutions generally include one or more of water (from about 90% to about 99%), preservative (from about 0.01% to about 0.5%), thickening agent (from 0% to about 5%), flavoring agent (from about 0.04% to about 2%), sweetening agent (from about 0.1% to about 3%), and surfactant (from 0% to about 5%).

Types of orally acceptable carrier materials or excipients which may be included in compositions of the present invention, along with specific non-limiting examples, are discussed in the following paragraphs.

### Desensitizing Agent

An optional active agent that may be added to the present compositions is a dentinal desensitizing agent to control hypersensitivity, such as salts of potassium, calcium, strontium and tin including nitrate, chloride, fluoride, phosphates, pyrophosphate, polyphosphate, citrate, oxalate and sulfate.

### Anticalculus Agent

The present compositions may optionally include an anticalculus agent, such as a pyrophosphate salt as a source of pyrophosphate ion. The pyrophosphate salts useful in the present compositions include the dialkali metal pyrophosphate salts, tetraalkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate (K₄P₂O₇) in their unhydrated as well as hydrated forms are the preferred species. In compositions of the present invention, the pyrophosphate salt may be present in one of three ways: predominately dissolved, predominately undissolved, or a mixture of dissolved and undissolved pyrophosphate.

Compositions comprising predominately dissolved pyrophosphate refer to compositions where at least one pyrophosphate ion source is in an amount sufficient to provide at least about 1.0% free pyrophosphate ions. The amount of free pyrophosphate ions may be from about 1% to about 15%, from about 1.5% to about 10% in one embodiment, and from about 2% to about 6% in another embodiment. Free pyrophosphate ions may be present in a variety of protonated states depending on the pH of the composition.

Compositions comprising predominately undissolved pyrophosphate refer to compositions containing no more than about 20% of the total pyrophosphate salt dissolved in the composition, or less than about 10% of the total pyrophosphate dissolved in the composition. Tetrasodium pyrophosphate salt is a preferred pyrophosphate salt in these compositions. Tetrasodium pyrophosphate may be the anhydrous salt form or the decahydrate form, or any other species stable in solid form in the dentifrice compositions. The salt is in its solid particle form, which may be its crystalline and/or amorphous state, with the particle size of the salt preferably being small enough to be aesthetically acceptable and readily soluble during use. The amount of pyrophosphate salt useful in making these compositions is any tartar control effective amount, generally from about 1.5% to about 15%, from about 2% to about 10%, or from about 3% to about 8%, by weight of the dentifrice composition.

Compositions may also comprise a mixture of dissolved and undissolved pyrophosphate salts. Any of the above mentioned pyrophosphate salts may be used.

The pyrophosphate salts are described in more detail in Kirk-Othmer Encyclopedia of Chemical Technology, Third Edition, Volume 17, Wiley-Interscience Publishers (1982).

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Pat. No. 4,627,977, to Gaffar et al., as well as, e.g., polyamino propane sulfonic acid (AMPS), diphosphonates (e.g., EHDP; AHP), polypeptides (such as polyaspartic and polyglutamic acids), and mixtures thereof.

### Fluoride Ion Source

It is common to have a water-soluble fluoride compound present in dentifrices and other oral compositions in an amount sufficient to give a fluoride ion concentration in the composition, and/or when it is used of from about 0.0025% to about 5.0% by weight or from about 0.005% to about 2.0% by weight, to provide anticaries effectiveness. A wide variety of fluoride ion-yielding materials can be employed as sources of soluble fluoride in the present compositions. Examples of suitable fluoride ion-yielding materials are found in U.S. Patent No. 3,535,421, October 20, 1970 to Briner et al. and U.S. Patent No. 3,678,154, July 18, 1972 to Widder et al. Representative fluoride ion sources include: stannous fluoride, sodium fluoride, potassium fluoride, sodium monofluorophosphate, indium fluoride, amine fluoride and many others. Stannous fluoride and sodium fluoride are among preferred sources, as well as mixtures thereof.

### Abrasives

Dental abrasives useful in the compositions of the subject invention include many different materials. The material selected must be one which is compatible within the composition of interest and does not excessively abrade dentin. Suitable abrasives include, for example, silicas including gels and precipitates, insoluble sodium polymetaphosphate, hydrated alumina, calcium carbonate, dicalcium orthophosphate dihydrate, calcium pyrophosphate, tricalcium phosphate, calcium polymetaphosphate, and resinous abrasive materials such as particulate condensation products of urea and formaldehyde.

Another class of abrasives for use in the present compositions is the particulate thermosetting polymerized resins as described in U.S. Pat. No. 3,070,510 issued to Cooley & Grabenstetter on Dec. 25, 1962. Suitable resins include, for example, melamines, phenolics, ureas, melamine-ureas, melamine-formaldehydes, urea-formaldehyde, melamine-urea-formaldehydes, cross-linked epoxides, and cross-linked polyesters.

Silica dental abrasives of various types are preferred because of their unique benefits of exceptional dental cleaning and polishing performance without unduly abrading tooth enamel or dentine. The silica abrasive polishing materials herein, as well as other abrasives, generally have an average particle size ranging between about 0.1 to about 30 microns, and preferably from about 5 to about 15 microns. The abrasive can be precipitated silica or silica gels such as the silica xerogels described in Pader et al., U.S. Patent 3,538,230, issued Mar. 2, 1970, and DiGiulio, U.S. Patent 3,862,307, issued Jan. 21, 1975. Examples include the silica xerogels marketed under the trade name "Syloid" by the W.R. Grace & Company, Davison Chemical Division and precipitated silica materials such as those marketed by the J. M. Huber Corporation under the trade name, Zeodent®, particularly the silicas carrying the designation Zeodent® 119, Zeodent® 118, Zeodent® 109 and Zeodent® 129. The types of silica dental abrasives useful in the toothpastes of the present invention are described in more detail in Wason, U.S. Patent 4,340,583; and in commonly-assigned US Pat. Nos. 5,603,920; 5,589,160; 5,658,553; 5,651,958; and 6,740,311.

Mixtures of abrasives can be used such as mixtures of the various grades of Zeodent® silica abrasives listed above. The total amount of abrasive in dentifrice compositions of the subject invention typically range from about 6% to about 70% by weight; toothpastes generally contain from about 10% to about 50% of abrasives, by weight of the composition. Dental solution, mouth spray, mouthwash and non-abrasive gel compositions of the subject invention typically contain little or no abrasive.

### Tooth Substantive Agent

The present invention may include a tooth substantive agent such as polymeric surface active agents (PMSA's), which are polyelectrolytes, more specifically anionic polymers. The PMSA's contain anionic groups, e.g., phosphate, phosphonate, carboxy, or mixtures thereof, and thus, have the capability to interact with cationic or positively charged entities. The "mineral" descriptor is intended to convey that the surface activity or substantivity of the polymer is toward mineral surfaces such as calcium phosphate minerals or teeth.

PMSA's are useful in the present compositions because of their stain prevention benefit. It is believed the PMSA's provide a stain prevention benefit because of their reactivity or substantivity to mineral surfaces, resulting in desorption of portions of undesirable adsorbed pellicle proteins, in particular those associated with binding color bodies that stain teeth, calculus development and attraction of undesirable microbial species. The retention of these PMSA's on teeth can also prevent stains from accruing due to disruption of binding sites of color bodies on tooth surfaces.

The ability of PMSA's to bind stain promoting ingredients of oral care products, for example, stannous ions and cationic antimicrobials, is also believed to be helpful. The PMSA will also provide tooth surface conditioning effects which produce desirable effects on surface thermodynamic properties and surface film properties, which impart improved clean feel aesthetics both during and most importantly, following rinsing or brushing. Many of these polymeric agents are also known or expected to provide tartar control benefits when applied in oral compositions, hence providing improvement in both the appearance of teeth and their tactile impression to consumers.

The polymeric mineral surface active agents include an agent which will have a strong affinity for the tooth surface, deposit a polymer layer or coating on the tooth surface and produce the desired surface modification effects. Suitable examples of such polymers are polyelectrolytes such as condensed phosphorylated polymers; polyphosphonates; copolymers of phosphate- or phosphonate-containing monomers or polymers with other monomers such as ethylenically unsaturated monomers and amino acids or with other polymers such as proteins, polypeptides, polysaccharides, poly(acrylate), poly(acrylamide), poly(methacrylate), poly(ethacrylate), poly(hydroxyalkylmethacrylate), poly(vinyl alcohol), poly(maleic anhydride), poly(maleate) poly(amide), poly(ethylene amine), poly(ethylene glycol), poly(propylene glycol), poly(vinyl acetate) and poly(vinyl benzyl chloride); polycarboxylates and carboxy-substituted polymers; and mixtures thereof. Suitable polymeric mineral surface active agents include the carboxy-substituted alcohol polymers described in U.S. Patent Nos. 5,292,501; 5,213,789, 5,093,170; 5,009,882; and 4,939,284; all to Degenhardt et al. and the diphosphonate-derivatized polymers in U.S. patent 5,011,913 to Benedict et al; the synthetic anionic polymers including polyacrylates and copolymers of maleic anhydride or acid and methyl vinyl ether (e.g., Gantrez), as described, for example, in U.S. Patent 4,627,977, to Gaffar et al. Diphosphonate modified polyacrylic acid is another example. Polymers with activity must have sufficient surface binding propensity to desorb pellicle proteins and remain affixed to enamel surfaces. For tooth surfaces, polymers with end or side chain phosphate or phosphonate functions are preferred although other polymers with mineral binding activity may prove effective depending upon adsorption affinity.

Additional examples of suitable phosphonate containing polymeric mineral surface active agents include the geminal diphosphonate polymers disclosed as anticalculus agents in US 4,877,603 to Degenhardt et al; phosphonate group containing copolymers disclosed in US 4,749,758 to Dursch et al. and in GB 1,290,724 (both assigned to Hoechst) suitable for use in detergent and cleaning compositions; and the copolymers and cotelomers disclosed as useful for applications including scale and corrosion inhibition, coatings, cements and ion-exchange resins in US 5,980,776 to Zakikhani et al. and US 6,071,434 to Davis et al. Additional polymers include the water-soluble copolymers of vinylphosphonic acid and acrylic acid and salts thereof disclosed in GB 1,290,724 wherein the copolymers contain from about 10% to about 90% by weight vinylphosphonic acid and from about 90% to about 10% by weight acrylic acid, more particularly wherein the copolymers have a weight ratio of vinylphosphonic acid to acrylic acid of 70% vinylphosphonic acid to 30% acrylic acid; 50% vinylphosphonic acid to 50% acrylic acid; or 30% vinylphosphonic acid to 70% acrylic acid. Other suitable polymers include the water soluble polymers disclosed by Zakikhani and Davis prepared by copolymerizing diphosphonate or polyphosphonate monomers having one or more unsaturated C=C bonds (e.g., vinylidene-1,1-diphosphonic acid and 2-(hydroxyphosphinyl)ethylidene-1,1-diphosphonic acid), with at least one further compound having unsaturated C=C bonds (e.g., acrylate and methacrylate monomers). Suitable polymers include the diphosphonate/acrylate polymers supplied by Rhodia under the designation ITC 1087 (Average MW 3000-60,000) and Polymer 1154 (Average MW 6000-55,000).

Suitable PMSA's will be stable with other components of the oral care composition such as ionic fluoride and metal ions, and are stable to hydrolysis in high water content formulations, thus permitting a simple single phase dentifrice or mouthrinse formulation. If the PMSA does not have these stability properties, one option is a dual phase formulation with the PMSA separated from the fluoride or other incompatible component. Another option is to formulate non-aqueous, essentially non-aqueous or limited water compositions to minimize reaction between the PMSA and other components.

A preferred PMSA is a polyphosphate. A polyphosphate is generally understood to consist of two or more phosphate molecules arranged primarily in a linear configuration, although some cyclic derivatives may be present. Although pyrophosphates (n=2) are technically polyphosphates, the polyphosphates desired are those having around three or more phosphate groups so that surface adsorption at effective concentrations produces sufficient non-bound phosphate functions, which enhance the anionic surface charge as well as hydrophilic character of the surfaces. The polyphosphate salts desired include tripolyphosphate, tetrapolyphosphate and hexametaphosphate, among others. Polyphosphates larger than tetrapolyphosphate usually occur as amorphous glassy materials. Preferred in this invention are the linear polyphosphates having the formula: XO(XPO₃)ₙX, wherein X is sodium, potassium or ammonium and n averages from about 3 to about 125. Preferred polyphosphates are those having n averaging from about 6 to about 21, such as those commercially known as Sodaphos (n≈6), Hexaphos (n≈13), and Glass H (n≈21) and manufactured by FMC Corporation and Astaris. These polyphosphates may be used alone or in combination. Some polyphosphates are susceptible to hydrolysis in high water formulations at acid pH, particularly below pH 5. Thus it is preferred to use longer-chain polyphosphates, such as Glass H having an average chain length of about 21. Such longer-chain polyphosphates when undergoing hydrolysis, produce shorter-chain polyphosphates which are still effective to deposit onto teeth and provide a stain preventive benefit.

Other polyphosphorylated compounds may be used in addition to or instead of the polyphosphate, in particular polyphosphorylated inositol compounds such as phytic acid, myo-inositol pentakis(dihydrogen phosphate); myo-inositol tetrakis(dihydrogen phosphate), myo-inositol trikis(dihydrogen phosphate), and an alkali metal, alkaline earth metal or ammonium salt thereof. Preferred herein is phytic acid, also known as myo-inositol 1,2,3,4,5,6-hexakis (dihydrogen phosphate) or inositol hexaphosphoric acid, and its alkali metal, alkaline earth metal or ammonium salts. Herein, the term "phytate" includes phytic acid and its salts as well as the other polyphosphorylated inositol compounds.

The amount of tooth substantive agent will typically be from about 0.1% to about 35% by weight of the total oral composition. In dentifrice formulations, the amount is typically from about 2% to about 30%, from about 5% to about 25%, or from about 6% to about 20%. In mouthrinse compositions, the amount of tooth substantive agent is typically from about 0.1% to 5 % or from about 0.5% to about 3 %.

In addition to creating surface modifying effects, the tooth substantive agent may also function to solubilize insoluble salts. For example, Glass H has been found to solubilize insoluble stannous salts. Thus, in compositions containing stannous fluoride for example, Glass H contributes to decreasing the stain promoting effect of stannous.

### Chelating agents

Another optional agent is a chelating agent, also called sequestrants, such as gluconic acid, tartaric acid, citric acid and pharmaceutically-acceptable salts thereof. Chelating agents are able to complex calcium found in the cell walls of the bacteria. Chelating agents can also disrupt plaque by removing calcium from the calcium bridges which help hold this biomass intact. However, it is not desired to use a chelating agent which has an affinity for calcium that is too high, as this may result in tooth demineralization, which is contrary to the objects and intentions of the present invention. Suitable chelating agents will generally have a calcium binding constant of about 10¹ to 10⁵ to provide improved cleaning with reduced plaque and calculus formation. Chelating agents also have the ability to complex with metallic ions and thus aid in preventing their adverse effects on the stability or appearance of products. Chelation of ions, such as iron or copper, helps retard oxidative deterioration of finished products.

Examples of suitable chelating agents are sodium or potassium gluconate and citrate; citric acid/alkali metal citrate combination; disodium tartrate; dipotassium tartrate; sodium potassium tartrate; sodium hydrogen tartrate; potassium hydrogen tartrate; sodium, potassium or ammonium polyphosphates and mixtures thereof. The amounts of chelating agent suitable for use in the present invention will typically be from about 0.1% to about 2.5%, from about 0.5% to about 2.5%, or from about 1.0% to about 2.5%.

Still other chelating agents suitable for use in the present invention are the anionic polymeric polycarboxylates. Such materials are well known in the art, being employed in the form of their free acids or partially or preferably fully neutralized water soluble alkali metal (e.g. potassium and preferably sodium) or ammonium salts. Examples are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 1,000,000. These copolymers are available for example as Gantrez AN 139 (M.W. 500,000), AN 119 (M.W. 250,000) and S-97 Pharmaceutical Grade (M.W. 70,000), of GAF Chemicals Corporation.

Other operative polymeric polycarboxylates include the 1:1 copolymers of maleic anhydride with ethyl acrylate, hydroxyethyl methacrylate, N-vinyl-2-pyrrolidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Additional operative polymeric polycarboxylates are disclosed in U.S. Pat. Nos. 4,138,477 and 4,183,914 to Gaffar et al. and include copolymers of maleic anhydride with styrene, isobutylene or ethyl vinyl ether; polyacrylic, polyitaconic and polymaleic acids; and sulfoacrylic oligomers of M.W. as low as 1,000 available as Uniroyal ND-2.

### Surfactants

The present compositions may also comprise surfactants, also commonly referred to as sudsing agents. Suitable surfactants are those which are reasonably stable and foam throughout a wide pH range. The surfactant may be anionic, nonionic, amphoteric, zwitterionic, cationic, or mixtures thereof.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate (SLS) and sodium coconut monoglyceride sulfonates are examples of anionic surfactants of this type. Other suitable anionic surfactants are sarcosinates, such as sodium lauroyl sarcosinate, taurates, sodium lauryl sulfoacetate, sodium lauroyl isethionate, sodium laureth carboxylate, and sodium dodecyl benzenesulfonate. Mixtures of anionic surfactants can also be employed. Many suitable anionic surfactants are disclosed by Agricola et al., U.S. Patent 3,959,458, issued May 25, 1976. The present composition typically comprises an anionic surfactant at a level of from about 0.025% to about 9%, from about 0.05% to about 5% in some embodiments, and from about 0.1 % to about 1% in other embodiments.

Another suitable surfactant is one selected from the group consisting of sarcosinate surfactants, isethionate surfactants and taurate surfactants. Preferred for use herein are alkali metal or ammonium salts of these surfactants, such as the sodium and potassium salts of the following: lauroyl sarcosinate, myristoyl sarcosinate, palmitoyl sarcosinate, stearoyl sarcosinate and oleoyl sarcosinate. The sarcosinate surfactant may be present in the compositions of the present invention from about 0.1% to about 2.5% or from about 0.5% to about 2.0% by weight of the total composition.

Cationic surfactants useful in the present invention include derivatives of aliphatic quaternary ammonium compounds having one long alkyl chain containing from about 8 to 18 carbon atoms such as lauryl trimethylammonium chloride; cetyl pyridinium chloride; cetyl trimethylammonium bromide; di-isobutylphenoxyethyl-dimethylbenzylammonium chloride; coconut alkyltrimethylammonium nitrite; cetyl pyridinium fluoride; etc. The quaternary ammonium fluorides having detergent properties are described in U.S. Patent 3,535,421 to Briner et al. Certain cationic surfactants can also act as germicides in the compositions disclosed herein. Cationic surfactants such as chlorhexidine, although suitable for use in the current invention, may not be preferred due to their capacity to stain the oral cavity's hard tissues. Persons skilled in the art are aware of this possibility and should incorporate cationic surfactants with this limitation in mind.

Nonionic surfactants that can be used in the compositions of the present invention include compounds produced by the condensation of alkylene oxide groups (hydrophilic in nature) with an organic hydrophobic compound which may be aliphatic or alkylaromatic in nature. Examples of suitable nonionic surfactants include the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

Zwitterionic synthetic surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate.

Suitable betaine surfactants are disclosed in U.S. Patent 5,180,577 to Polefka et al., issued January 19, 1993. Typical alkyl dimethyl betaines include decyl betaine or 2-(N-decyl-N,N-dimethylammonio) acetate, coco betaine or 2-(N-coc-N, N-dimethyl ammonio) acetate, myristyl betaine, palmityl betaine, lauryl betaine, cetyl betaine, cetyl betaine, stearyl betaine, etc. The amidobetaines are exemplified by cocoamidoethyl betaine, cocoamidopropyl betaine, lauramidopropyl betaine and the like. The betaines of choice include cocoamidopropyl betaines such as lauramidopropyl betaine.

### Thickening Agents

In preparing toothpaste or gels, thickening agents are added to provide a desirable consistency to the composition, to provide desirable active release characteristics upon use, to provide shelf stability, and to provide stability of the composition, etc. Suitable thickening agents include one or a combination of carboxyvinyl polymers, carrageenan, hydroxyethyl cellulose (HEC), natural and synthetic clays (e.g., Veegum and laponite) and water soluble salts of cellulose ethers such as sodium carboxymethylcellulose (CMC) and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture.

Suitable carboxyvinyl polymers useful as thickening or gelling agents include carbomers which are homopolymers of acrylic acid crosslinked with an alkyl ether of pentaerythritol or an alkyl ether of sucrose. Carbomers are commercially available from B.F. Goodrich as the Carbopol® series, including Carbopol 934, 940, 941, 956, and mixtures thereof.

Thickening agents are typically present in an amount from about 0.1% to about 15%, from about 2% to about 10%, or from about 4% to about 8%, by weight of the total toothpaste or gel composition, can be used. Higher concentrations may be used for chewing gums, lozenges and breath mints, sachets, non-abrasive gels and subgingival gels.

### Humectants

Another optional carrier material of the present compositions is a humectant. The humectant serves to keep toothpaste compositions from hardening upon exposure to air, to give compositions a moist feel to the mouth, and, for particular humectants, to impart desirable sweetness of flavor to toothpaste compositions. The humectant, on a pure humectant basis, generally comprises from about 0% to about 70% or from about 5% to about 25%, by weight of the compositions herein. Suitable humectants for use in compositions of the subject invention include edible polyhydric alcohols such as glycerin, sorbitol, xylitol, butylene glycol, polyethylene glycol, propylene glycol and trimethyl glycine.

### Flavor System

A flavor system is typically added to oral care compositions, to provide a pleasant tasting composition and to effectively mask any unpleasant taste and sensations due to certain components of the composition such as antimicrobial actives or peroxide. Pleasant tasting compositions improve user compliance to prescribed or recommended use of oral care products. The present flavor system will comprise flavor components, such as those that have been found to be relatively stable in the presence of usual oral care product actives, carrier materials or excipients. The flavor system may comprise flavor ingredients including but not limited to peppermint oil, corn mint oil, spearmint oil, oil of wintergreen, clove bud oil, cassia, sage, parsley oil, marjoram, lemon, lime, orange, *cis*-jasmone, 2,5-dimethyl-4-hydroxy-3(2H)-furanone, 5-ethyl-3-hydroxy-4-methyl-2(5H)-furanone, vanillin, ethyl vanillin, 2-methoxybenzaldehyde, benzaldehyde; cinnamaldehyde, hexyl cinnamaldehyde, alpha-methyl cinnamaldehyde, ortho-methoxy cinnamaldehyde, alpha-amyl cinnamaldehydepropenyl guaethol, heliotropine, 4-*cis-*heptenal, diacetyl, methyl-ρ-tert-butyl phenyl acetate, menthol, methyl salicylate, ethyl salicylate, 1-menthyl acetate, oxanone, alpha-irisone, methyl cinnamate, ethyl cinnamate, butyl cinnamate, ethyl butyrate, ethyl acetate, methyl anthranilate, *iso*-amyl acetate, iso-amyl butyrate, allyl caproate, eugenol, eucalyptol, thymol, cinnamic alcohol, octanol, octanal, decanol, decanal, phenylethyl alcohol, benzyl alcohol, alpha-terpineol, linalool, limonene, citral, maltol, ethyl maltol, anethole, dihydroanethole, carvone, menthone, β-damascenone, ionone, gamma decalactone, gamma nonalactone, gamma undecalactone and mixtures thereof. Generally suitable flavoring ingredients are those containing structural features and functional groups that are less prone to redox reactions. These include derivatives of flavor chemicals that are saturated or contain stable aromatic rings or ester groups. Also suitable are flavor chemicals that may undergo some oxidation or degradation without resulting in a significant change in the flavor character or profile. The flavor ingredients may be supplied in the composition as single or purified chemicals or by addition of natural oils or extracts that have preferably undergone a refining treatment to remove components that are relatively unstable and may degrade and alter the desired flavor profile, resulting in a less acceptable product from an organoleptic standpoint. Flavoring agents are generally used in the compositions at levels of from about 0.001% to about 5%, by weight of the composition.

The flavor system will typically include a sweetening agent. Suitable sweeteners include those well known in the art, including both natural and artificial sweeteners. Some suitable water-soluble sweeteners include monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin. Suitable water-soluble artificial sweeteners include soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like. Other suitable sweeteners include dipeptide based sweeteners, such as L-aspartic acid derived sweeteners, such as L-aspartyl-L-phenylalanine methyl ester (aspartame) and materials described in U.S. Pat. No. 3,492,131, L-alpha-aspartyl-N-(2,2,4,4-tetramethyl-3-thietanyl)-D-alaninamide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclohexylen)-alanine, and the like. Water-soluble sweeteners derived from naturally occurring water-soluble sweeteners, such as a chlorinated derivative of ordinary sugar (sucrose), known, for example, under the product description of sucralose as well as protein based sweeteners such as thaumatoccous danielli (Thaumatin I and II) can be used. A composition preferably contains from about 0.1% to about 10% of sweetener, by weight.

Suitable cooling agents or coolants include a wide variety of materials such as menthol and derivatives thereof. Among synthetic coolants, many are derivatives of or are structurally related to menthol, i.e., containing the cyclohexane moiety, and derivatized with functional groups including carboxamide, ketal, ester, ether and alcohol. Examples include the *ρ-*menthanecarboxamide compounds such as N-ethyl-*p*-menthan-3-carboxamide, known commercially as "WS-3", and others in the series such as WS-5, WS-11, WS-14 and WS-30. An example of a synthetic carboxamide coolant that is structurally unrelated to menthol is N,2,3-trimethyl-2-isopropylbutanamide, known as "WS-23". Additional suitable coolants include 3-1-menthoxypropane-1,2-diol known as TK-10, isopulegol (under the tradename Coolact P) and ρ-menthane-3,8-diol (under the tradename Coolact 38D) all available from Takasago; menthone glycerol acetal known as MGA; menthyl esthers such as menthyl acetate, menthyl acetoacetate, menthyl lactate known as Frescolat® supplied by Haarmann and Reimer, and monomenthyl succinate under the tradename Physcool from V. Mane. The terms menthol and menthyl as used herein include dextro- and levorotatory isomers of these compounds and racemic mixtures thereof. TK-10 is described in U.S. Pat. No. 4,459,425, Amano et al. WS-3 and other carboxamide cooling agents are described for example in U.S. Pat. Nos. 4,136,163; 4,150,052; 4,153,679; 4,157,384; 4,178,459 and 4,230,688. Additional N-substituted *ρ*-menthane carboxamides are described in WO 2005/049553A1 including N-(4-cyanomethylphenyl)-*ρ-*menthanecarboxamide, N-(4-sulfamoylphenyl)-*ρ*-menthanecarboxamide, N-(4-cyanophenyl)-*ρ-*menthanecarboxamide, N-(4-acetylphenyl)-*ρ*-menthanecarboxamide, N-(4-hydroxymethylphenyl)-p-menthanecarboxamide and N-(3-hydroxy-4-methoxyphenyl)-*ρ-*menthanecarboxamide.

In addition the flavor system may include salivating agents, hydration and moisturization agents, warming agents, and numbing agents. These agents are present in the compositions at a level of from about 0.001% to about 10% or from about 0.1% to about 1%, by weight of the composition. Suitable salivating agents include Jambu® manufactured by Takasago and Optaflow® from Symrise. Examples of hydration agents include polyols such as erythritol. Suitable numbing agents include benzocaine, lidocaine, clove bud oil, and ethanol. Examples of warming agents include ethanol, capsicum and nicotinate esters, such as benzyl nicotinate.

### Miscellaneous Carrier Materials

Water employed in the preparation of commercially suitable oral compositions desirably would be of low ion content and free of organic impurities. Water may comprise up to about 99% by weight of the aqueous compositions herein. These amounts of water include the free water which is added plus that which is introduced with other materials, such as with sorbitol.

The present invention may also include an alkali metal bicarbonate salt, which may serve a number of functions including abrasive, deodorant, buffering and adjusting pH. Alkali metal bicarbonate salts are soluble in water and unless stabilized, tend to release carbon dioxide in an aqueous system. Sodium bicarbonate, also known as baking soda, is a commonly used alkali metal bicarbonate salt. The present composition may contain from about 0.5% to about 30%, from about 0.5% to about 15% or from about 0.5% to about 5% of an alkali metal bicarbonate.

The pH of the present compositions may be adjusted through the use of buffering agents. Buffering agents, as used herein, refer to agents that can be used to adjust the pH of aqueous compositions such as mouthrinses and dental solutions typically to a range of about pH 4.0 to about pH 8.0. Buffering agents include sodium bicarbonate, monosodium phosphate, trisodium phosphate, sodium hydroxide, sodium carbonate, sodium acid pyrophosphate, citric acid, and sodium citrate. Buffering agents are typically included at a level of from about 0.5% to about 10%, by weight of the present compositions.

Poloxamers may be employed in the present compositions. A poloxamer is classified as a nonionic surfactant and may also function as an emulsifying agent, binder, stabilizer, and other related functions. Poloxamers are difunctional block-polymers terminating in primary hydroxyl groups with molecular weights ranging from 1,000 to above 15,000. Poloxamers are sold under the tradename of Pluronics and Pluraflo by BASF. Suitable poloxamers for this invention are Poloxamer 407 and Pluraflo L4370.

Other emulsifying agents that may be used in the present compositions include polymeric emulsifiers such as the Pemulen® series available from B.F. Goodrich, and which are predominantly high molecular weight polyacrylic acid polymers useful as emulsifiers for hydrophobic substances.

Titanium dioxide may also be added to the present composition. Titanium dioxide is a white powder which adds opacity to the compositions. Titanium dioxide generally comprises from about 0.25% to about 5% by weight of dentifrice compositions.

Other optional agents that may be used in the present compositions include dimethicone copolyols selected from alkyl- and alkoxy-dimethicone copolyols, such as C12 to C20 alkyl dimethicone copolyols and mixtures thereof. Highly preferred is cetyl dimethicone copolyol marketed under the trade name Abil EM90. The dimethicone copolyol is generally present in a level of from about 0.01% to about 25%, from about 0.1% to about 5%, or from about 0.5% to about 1.5% by weight. The dimethicone copolyols aid in providing positive tooth feel benefits.

### Method of Use

The present invention also relates to the use of the compositions for control of whole mouth malodor and for controlling bacterial activity in the oral which cause undesirable conditions including plaque, caries, calculus, gingivitis, and periodontal disease. The benefits of these compositions may increase over time when the composition is used repeatedly.

The method of use or treatment herein comprises contacting a subject's dental enamel surfaces and mucosa in the mouth with the oral compositions according to the present invention. The method may comprise brushing with a dentifrice or rinsing with a dentifrice slurry or mouthrinse. Other methods include contacting the topical oral gel, denture product, mouthspray, or other form with the subject's teeth and oral mucosa. The subject may be any person or animal whose tooth surface contact the oral composition. By animal is meant to include household pets or other domestic animals, or animals kept in captivity.

For example, a method of treatment may include a person brushing a dog's teeth with one of the dentifrice compositions. Another example would include rinsing a cat's mouth with an oral composition for a sufficient amount of time to see a benefit. Pet care products such as chews and toys may be formulated to contain the present oral compositions. The composition may be incorporated into a relatively supple but strong and durable material such as rawhide, ropes made from natural or synthetic fibers, and polymeric articles made from nylon, polyester or thermoplastic polyurethane. As the animal chews, licks or gnaws the product, the incorporated
active elements are released into the animal's oral cavity into a salivary medium, comparable to an effective brushing or rinsing.

### EXAMPLES

The following examples further describe and demonstrate embodiments within the scope of the present invention. These examples are given solely for the purpose of illustration and are not to be construed as limitations of the present invention as many variations thereof are possible without departing from the spirit and scope.

### Example I Dentifrice Compositions

Dentifrice composition Ib is according to the present invention Ia and Ic are shown as comparative examples with amounts of components in weight %. These compositions are made using conventional methods.

| Component | Ia | Ib | Ic |
|---|---|---|---|
| Water purified | Q.S. | Q.S. | Q.S. |
| Zinc citrate dihydrate | 0.788 | 0.788 | 0.533 |
| Sodium Citrate tribasic dihydrate | 0.274 | 0.274 | |
| Sodium Gluconate | | | 1.06 |
| Sodium Fluoride | 0.243 | 0.243 | 0.243 |
| Stannous Chloride | 0.209 | 0.209 | 1.16 |
| Anisaldehyde | 0.01-0.1 | 0.01-0.1 | 0.01-0.1 |
| β-Ionone | | 0.01-0.1 | |
| Sorbitol 70% | 40.5 | 40.5 | 38.0 |
| Sodium Saccharin | 0.3 | 0.3 | 0.5 |
| Hydroxyethyl cellulose | 0.3 | 0.3 | 0.5 |
| Sodium CMC | 1.3 | 1.3 | 1.3 |
| Carrageenan Mixture | 0.7 | 0.7 | 0.7 |
| Titanium Dioxide | 0.525 | 0.525 | 0.525 |
| Hydrated Silica amorphous | 17.0 | 17.0 | 17.0 |
| Sodium Alkyl Sulfate 28% soln. | 5.0 | 5.0 | 5.0 |
| Sodium Hydroxide 50% soln. | | | 1.15 |
| Phytic Acid 50% soln. | | | 0.8 |
| Flavor | 1.1 | 1.1 | 1.1 |
| Color | | | 0.3 |
| | 100.0 | 100.0 | 100.0 |

### Example II Mouthrinse Compositions

Mouthrinse compositions (IIb and IIe,) according to the present invention made using conventional methods are shown below with amounts of components in weight %. IIa, IIc, IId and IIf are given as comparative examples.

| Component | IIa | IIb | IIc | IId | IIe | IIf |
|---|---|---|---|---|---|---|
| Purified Water | Q.S. | Q.S. | Q.S. | Q.S. | Q.S. | Q.S. |
| Glycerin | 6.0 | 6.0 | 6.0 | 6.0 | 6.0 | 6.0 |
| Alcohol | 9.0 | 9.0 | 5.0 | 9.0 | 9.0 | 9.0 |
| Flavor | 0.174 | 0.174 | 0.174 | 0.174 | 0.174 | 0.174 |
| Sodium Saccharin | 0.06 | 0.06 | 0.06 | 0.06 | 0.05 | 0.06 |
| Anisaldehyde | 0.01-0.1 | 0.01-0.1 | 0.01-0.1 | | 0.01-0.1 | 0.01-0.1 |
| 4-isopropylbenzaldehyde | | | 0.01-0.1 | 0.01-0.1 | | |
| 1-(4-Methoxy-phenyl)-2-methyl-propan-1-one | | | | 0.01-0.1 | | |
| β-ionone | | 0.01-0.1 | | | 0.01-0.1 | |
| Cetylpyridinum Chloride | 0.045 | 0.045 | | 0.045 | | 0.045 |
| Zinc Lactate dihydrate | 0.1 | 0.1 | 0.1 | 0.1 | 0.1 | |
| Sodium fluoride | | | | | 0.63 | |
| Polysorbate 80 | 0.12 | 0.12 | 0.120 | 0.12 | 0.12 | 0.12 |
| Poloxamer 407 | 0.08 | 0.08 | 0.05 | 0.05 | 0.05 | 0.05 |
| Sodium Benzoate | 0.054 | 0.054 | 0.054 | 0.054 | 0.054 | 0.054 |
| Benzoic Acid | 0.005 | 0.005 | 0.005 | 0.005 | 0.005 | 0.005 |
| Total | 100.0 | 100.0 | 100.0 | 100.0 | 100.0 | 100.0 |

### Example III Demonstration Kits

In a further aspect the present invention provides for a method and kit for demonstrating and comparing the effectiveness of oral care compositions such as mouthrinses and dentifrices for controlling food odors such as garlic and onion. For example, a kit for use with consumers was prepared as follows and used to demonstrate the effectiveness of a mouthrinse according to the present invention in eliminating garlic and onion odor compared to a water rinse.
- Prepare model odor samples to represent bad food odors, such as a 30/70 solution of garlic/onion, by mixing together commercially available Howard's Garlic Juice and Howard's Onion Juice. As an alternative a synthetic garlic stock comprising a mixture of five sulfur compounds (ethanethiol, 1-propanethiol, dimethyl disulfide, methyl allyl disulfide and dimethyl disulfide) or a minced garlic water mixture may be used to simulate a garlic odor.
- Prepare model odor sample vials by filling vials with an amount of the 30/70 garlic/onion solution and capping filled vials immediately to seal and prevent contamination and loss of volatile sulfur compound odorants (VSC's). Typically about 0.1 ml of the above solution is sufficient to simulate a mouth malodor such as associated with consumption of garlic and/or onion. An Eppendorf Repeater Pipette Plus may be used to measure out samples for filling the vials.
- Prepare control product vials by filling vials with an amount of sterile water (from 5 ml to 30 ml, typically 10 ml to 15 ml representing mouthrinse use levels) and capping filled vials immediately to seal and prevent contamination.
- Prepare test product vials by filling vials with test mouthrinse product (same amount as control) and capping filled vials immediately to seal and prevent contamination.
- Prepare waste vials for emptying used products and odor samples.
- Label all vials accordingly (model odor sample, test product, control product and waste vial) with instructions on how to use during the demonstration.
- Assemble labeled vials into a demo kit with instructions for conducting the demonstration with consumers or panelists.

The demonstration usage protocol is described below.
- Pour Water Control vial contents into an Odor Sample vial, cap the vial and shake for 30 seconds.
- After shaking, dispose contents of the Odor Sample + Water Control vial ("Control Vial") into a waste vial and recap the emptied Control Vial
- Have consumer/panelist uncap and smell the emptied Control Vial.
- Pour Test Mouthrinse vial contents into an Odor Sample vial, cap the vial and shake for 30 seconds.
- After shaking, dispose contents of the Odor Sample +Test Mouthrinse vial ("Test Vial") into a waste vial and recap the emptied Test Vial
- Have consumer/panelist uncap and smell the emptied Test Vial.

The demo kit and demonstration protocol were used in a blind test among 75 panelists who were each asked to evaluate a control sample and a test sample. An emptied Control Vial was presented first to each panelist to represent mouth/breath odor status after consumption of garlic/onion, followed by a water rinse treatment. An emptied Test Vial was presented next to the panelist, representing mouth/breath odor status after consumption of garlic/onion followed by gargling with a mouthrinse for 30 seconds and expectorating.

Panelists were asked to compare the odor they experienced after treatment of the model odor sample with the control and test samples. In this test, 100% of the panelists responded that the test mouthrinse (Example IIa above) dramatically reduced the bad garlic/onion odor, demonstrating the effectiveness of the present composition in reducing VSC's from garlic and onion. Headspace analysis for VSC's confirms the subjective evaluation.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An oral care composition comprising
(a) from 0.01% to 5.0%, by weight of the composition of an antibacterial agent comprising one or a mixture of a stannous ion source; a zinc ion source; or a copper ion source,
(b) from 0.01% to 0.2% by weight of a deodorizing or odor-neutralizing agent, wherein the deodorizing or odor-neutralizing agent comprises 4-methoxybenzaldehyde (anisaldehyde) and
(c) an orally-acceptable carrier,
wherein the deodorizing or odor-neutralizing agent further comprises β-ionone as additional odor-neutralizing compoundat a level of from 0.01% to 0.1% by weight of the composition.

2. An oral care composition according to Claim 1, wherein the deodorizing or odor-neutralizing agent is present in an amount from 0.01% to 0.1% by weight of the composition.

3. An oral care composition according to any one of the preceding claims in a form selected from toothpaste, dentifrice, subgingival gel, mouthrinse, mouthspray, mousse, foam or whitening gel.

4. An oral care composition according to any one of the preceding claims wherein the orally-acceptable carrier comprises one or more materials selected from a fluoride ion source, an anticalculus agent, a desensitizing agent, a peroxide source, a tooth substantive agent, a surfactant, or a flavor system.

5. An oral care composition according to any one of the preceding claims wherein the composition further comprises dimethicone copolyol selected from alkyl- and alkoxy-dimethicone copolyols and mixtures thereof.

6. An oral care composition according to the preceding claim wherein the dimethicone copolyol is present in a level from 0.01% to 25%, preferably from 0.1% to 5%, more preferred from 0.5% to 1.5% by weight of the composition.

7. Oral composition according to any one of the preceding claims for use in a method for control of whole mouth malodor and for use in a method for controlling bacterial activity in the oral cavity.

## Patentansprüche

1. Mundpflegezusammensetzung, umfassend:
(a) von 0,01 Gew.-% bis 5,0 Gew.-% der Zusammensetzung eines antibakteriellen Mittels, umfassend eines oder eine Mischung von einer Zinnionenquelle; einer Zinkionenquelle; oder einer Kupferionenquelle,
(b) von 0,01 Gew.-% bis 0,2 Gew.-% desodorierendes oder geruchsneutralisierendes Mittel, wobei das desodorierende oder geruchneutralisierende Mittel 4-Methoxybenzaldehyd (Anisaldehyd) umfasst, und
(c) einen oral verträglichen Träger,
wobei das desodorierende oder geruchsneutralisierende Mittel ferner β-lonon als zusätzliche geruchsneutralisierende Verbindung in einem Anteil von 0,01 Gew.-% bis 0,1 Gew.-% der Zusammensetzung umfasst.

2. Mundpflegezusammensetzung nach Anspruch 1, wobei das desodorierende oder geruchsneutralisierende Mittel in einer Menge von 0,01 Gew.-% bis 0,1 Gew.-% der Zusammensetzung vorliegt.

3. Mundpflegezusammensetzung nach einem der vorstehenden Ansprüche in einer Form ausgewählt aus
Zahnpasta, Zahnputzmittel, Subgingivalgel, Mundspülung, Mundspray, Mousse, Schaum oder Aufhellungsgel.

4. Mundpflegezusammensetzung nach einem der vorstehenden Ansprüche, wobei der oral verträgliche Träger ein oder mehrere Materialien umfasst, ausgewählt aus einer Fluoridionenquelle, einem Antizahnsteinmittel, einem Desensibilisierungsmittel, einer Peroxidquelle, einem Zahnsubstantivmittel, einem Tensid oder einem Geschmackssystem.

5. Mundpflegezusammensetzung nach einem der vorstehenden Ansprüche, wobei die Zusammensetzung ferner Dimethiconcopolyol umfasst, ausgewählt aus Alkyl- und Alkoxydimethiconcopolyolen und Mischungen davon.

6. Mundpflegezusammensetzung nach dem vorstehenden Anspruch, wobei das Dimethiconcopolyol in einem Anteil von 0,01 Gew.-% bis 25 Gew.-%, vorzugsweise von 0,1 Gew.-% bis 5 Gew.-%, mehr bevorzugt 0,5 bis 1,5 Gew.-% der Zusammensetzung vorliegt.

7. Orale Zusammensetzung nach einem der vorstehenden Ansprüche zur Verwendung in einem Verfahren zur Kontrolle von Mundgeruch und zur Verwendung in einem Verfahren zur Kontrolle der bakteriellen Aktivität in der Mundhöhle.

## Revendications

1. Composition de soins bucco-dentaires comprenant
(a) de 0,01 % à 5,0 %, en poids de la composition, d'un agent antibactérien comprenant une ou un mélange d'une source d'ions stanneux ; une source d'ions zinc ; ou une source d'ions cuivre,
(b) de 0,01 % à 0,2 % en poids d'un agent désodorisant ou neutralisant les odeurs, dans lequel l'agent désodorisant ou neutralisant les odeurs comprend du 4-méthoxybenzaldéhyde (anisaldéhyde) et
(c) un véhicule oralement acceptable,
dans laquelle l'agent désodorisant ou neutralisant les odeurs comprend en outre de la β-ionone en tant que composé supplémentaire neutralisant les odeurs à un taux allant de 0,01 % à 0,1 % en poids de la composition.

2. Composition de soins bucco-dentaires selon la revendication 1, dans laquelle l'agent désodorisant ou neutralisant les odeurs est présent en une quantité allant de 0,01 % à 0,1 % en poids de la composition.

3. Composition de soins bucco-dentaires selon l'une quelconque des revendications précédentes sous une forme choisie
parmi une pâte dentifrice, un dentifrice, un gel subgingival, un bain de rinçage de bouche, un aérosol pour la bouche, une écume, une mousse ou un gel de blanchissement.

4. Composition de soins bucco-dentaires selon l'une quelconque des revendications précédentes, dans laquelle le véhicule oralement acceptable comprend un ou plusieurs matériaux choisis parmi une source d'ions fluorure, un agent antitartre, un agent désensibilisant, une source de peroxyde, un agent substantif sur les dents, un agent tensioactif ou un système d'arôme.

5. Composition de soins bucco-dentaires selon l'une quelconque des revendications précédentes, dans laquelle la composition comprend en outre un diméthicone copolyol choisi parmi des alkyl- et alcoxy-diméthicone copolyols et leurs mélanges.

6. Composition de soins bucco-dentaires selon la revendication précédente, dans laquelle le diméthicone copolyol est présent à un taux allant de 0,01 % à 25 %, de préférence de 0,1 % à 5 %, plus préférablement de 0,5 % à 1,5 % en poids de la composition.

7. Composition orale selon l'une quelconque des revendications précédentes, pour une utilisation dans un procédé de contrôle des mauvaises odeurs de la bouche entière et pour une utilisation dans un procédé de contrôle de l'activité bactérienne dans la cavité buccale.
